# EUROPEAN PATENT SPECIFICATION

(11) **EP 3 919 613 B1**
(45) Date of publication and mention of the grant of the patent: **07.08.2024**
(21) Application number: 20178584.7
(22) Date of filing: 05.06.2020
(51) Int. Cl.: C12N 7/02, C12N 15/864, B01D 15/36, B01D 15/38, B01D 61/14, C12N 9/22

(54) **ENHANCED PURIFICATION OF ADENO-ASSOCIATED VIRUS TO MORE EFFECTIVELY REMOVE CONTAMINATING DNA**
VERBESSERTE REINIGUNG VON ADENO-ASSOZIIERTEM VIRUS UM KONTAMINIERENDE DNA EFFEKTIVER ZU ENTFERNEN
PURIFICATION AMÉLIORÉE DE VIRUS ADÉNO-ASSOCIÉS POUR L'ÉLIMINATION PLUS EFFICACE D'ADN CONTAMINANTS

(43) Date of publication of application: 08.12.2021
(73) Proprietor: Sartorius BIA Separations d.o.o., 5270 Ajdovscina (SI)
(72) Inventor: Gagnon, Peter S., Las Vegas, NV 89101 (US); Leskovec, Maja, 5213 Kanal (SI); Prebil, Sara Drmota, 5280 Idrija (SI); Strancar, Ales, 5270 Ajdovscina (SI)
(74) Representative: Novagraaf International SA

(56) References cited:
- WO-A1-02/12455
- WO-A1-2010/148143
- WO-A1-2019/006390
- WO-A2-03/097797

## Description

The present invention discloses a method for reducing a contaminating DNA content of a preparation containing AAV capsids and contaminating DNA.

### Background

Adeno-associated virus (AAV) has become a popular delivery vehicle for therapeutic DNA in the field of human gene therapy. The virus is produced by cell culture methods, most commonly using mammalian or insect host cells. The cell culture is manipulated so that the desired therapeutic DNA plasmid resides inside the AAV capsids. The cell culture is harvested and the DNA-containing AAV capsids are purified with standard chromatography methods such as affinity chromatography, ion exchange chromatography, hydrophobic interaction chromatography and others.

As with all human-injectable therapeutic agents, safety requires that contaminating DNA be reduced to very low levels. This has proven to be more challenging than expected. Recently developed analytical technology suggests that the difficulty may arise in part from residual DNA bound to the exteriors of the AAV capsids. This is perhaps unsurprising since AAV capsids are known to bind strongly to cation exchangers. Strong binding to cation exchangers is an indicator that AAV capsid exteriors are endowed with a strong positive charge. DNA is electronegative and binds strongly to positively charged surfaces. Whatever the mechanism or mechanisms, these findings highlight the difficulty of removing DNA.

At present, it is common industry practice to treat AAV harvests by simple addition of DNase enzymes. DNA reduction by this method is unfortunately modest and does not solve the problem of reducing contaminating DNA to very low levels. It provides a secondary benefit of improving harvest filterability from hopeless to possible, but filter clogging remains a routine problem, requiring excessive filtration media and resulting in loss of AAV capsids. It also prevents processing methods like tangential flow filtration from being able to concentrate the harvest to a significant degree. Concentration factors up to 2 are sometimes possible but little more.

WO 02/12455 A1 discloses large-scale recombinant AAV production and purification.

WO 2010/148143 discloses methods for purification of recombinant AAV vectors.

WO 03/097797 discloses methods for adenovirus purification.

### Summary

A novel method has been developed that provides surprising enhancements for purification of AAV, especially with respect to reducing DNA contamination. Cell culture harvest or lysate is exposed to a positively charged surface or surfaces to bind DNA not associated with AAV particles. Treatment with the positively charged surface(s) achieves an initial reduction of DNA and facilitates tangential flow filtration (TFF) to a degree that permits concentration factors of 10-20 or more. Given that DNase treatment of cell harvests or lysates does not permit such improvements in filterability, it is surprising that solid phase extraction with positively charged surfaces is able to do so.

### Brief Description of The Invention

According to the method of the invention a contaminating DNA content is reduced in a preparation containing AAV capsids and contaminating DNA, the method comprising the steps of
a) Performing an extraction of DNA with a solid phase bearing positive charges at its surface said solid phase is contacted with the preparation at a pH of 7.0 ± 1.0, and a salt concentration of 10 mM to 200 mM yielding a first fraction,
(b) Diafiltering the first fraction by a first tangential flow filtration to obtain a second fraction,
(c) Treating the second fraction with DNase,
(d) Diafiltering the DNase treated second fraction obtained by step c) by a second tangential flow filtration to a buffer with pH of 7.0 ± 1.0, and a salt concentration of 10 mM to 20 mM to yield a third fraction, and optionally
(e) Concentrating the third fraction by tangential flow filtration before supplemental chromatography.

In an embodiment of the method of the invention the solid phase can be in the form of loose bulk particles or a flow-through device.

In another embodiment of the method of the invention the tangential flow filtration may be performed with membranes selected from the group consisting of hollow-fiber membranes, including single hollow fiber membranes or membranes in bundles; or flat membranes, including single membranes or membranes that are stacked or rolled into cylinders.

In yet another embodiment of the method of the invention the first filtration can be performed in a buffer which is formulated to facilitate DNA digestion by a DNase.

In still another embodiment of the method of the invention the tangential flow filtration membrane can have a porosity with a molecular weight cutoff in the range of 10 kDa to 300 kDa, or 30 kDa to 300 kDa, or 60 kDa to 300 kDa, or 100 kDa to 300 kDa, or 200 kDa to 300 kDa, or 250 kDa to 300 kDa, preferably with molecular weight cutoffs of 100 kDa or more.

In a further embodiment of the method of the invention the preparation containing AAV capsids and contaminating DNA may contain AAV particles selected from the group consisting of AAV serotype 1, 2, 3, 4, 5, 6, 7, 8, 9, 10, 11, or other serotypes, including recombinant serotypes with features of more than one serotype.

In yet another embodiment of the method of the invention a fraction obtainable after step d) or e) can further be worked-up along the following options (i) through (iii):
Option (i) A cation exchanger chromatography,
Option (ii) An affinity chromatography, or
Option (iii) An anion exchange chromatography.

In still another embodiment of the second aspect of the method of the invention in purification step (i) the fraction of step d) or e) is loaded on the cation exchanger chromatography material at a pH of 4 to 6 in particular about pH 5, followed by
- a re-equilibration of the cation exchanger chromatography material to about pH 3.5 ± 0.5,
- an elution is performed with a salt gradient, and
- a treatment with an anion exchange chromatography column is performed thereafter. This process yields e.g. AAV of clinical quality.

In another embodiment of the method of the invention in purification step (ii) the fraction of step d) or e) is loaded on the affinity chromatography material and after elution an anion exchange chromatography treatment is performed. This process yields e.g. purified AAV.

In yet another embodiment of the method of the invention in purification step (iii) the fraction of step d) or e) is subjected to an anion exchange chromatography column. This process yields e.g. AAV of research grade.

### Brief Description of The Figures

Figure 1 depicts an overview of the method of the invention.
Figure 2 depicts a cation exchange chromatography profile showing application of the method of the invention.
Figure 3 depicts analytical reduced SDS-polyacrylamide gel electrophoresis (PAGE) comparing standard conditions with the conditions of the method of the invention.
Figure 4 depicts affinity chromatography capture of AAV from cell lysate.
Figure 5 depicts analytical reduced SDS-PAGE of fractions as indicated from the affinity chromatogram in illustrated in Figure 4.

### Detailed Description of The Invention

The aspects of the invention are described in more detail by way of example using AAV as an entity to be purified. Likewise, other entities may be purified in an analogous way which is easily recognized by a skilled person in the art having read and understood the disclosure of the present invention.

It will be recognized by some persons of experience in the art that DNA might alternatively be removed from process solutions by adding positively charged soluble polymers in place of positively charged solid phase materials. This is potentially precarious from a clinical safety perspective and may alter biological behavior of the purified capsids in vitro, causing misinterpretation of results and potentially causing results that are not representative of the AAV but are instead caused by the positively charged polymers. They may also have the effect of shielding some DNA from lysis by DNase enzymes. DNA is known to bind aggressively to the exteriors of AAV capsids. If positively charged polymers are added to a preparation containing such AAV capsids, the positively charged polymers may create a coating on the capsid-exterior-associated DNA. Strong associations between DNA and positively charged proteins is known to interfere with DNase enzymes. Positively charged polymers could do so to a greater extent. To the extent that positively charged polymers remain associated with AAV capsids after purification, they could subsequently leach into in vitro samples or be released gradually into living subjects, including laboratory animals or humans. Positively charged polymers are all chemical irritants that cause inflammation. Given that purified AAV capsids are ultimately to be administered to persons who are already sick, inadvertently dosing them with a known class of inflammatory agents represents an unnecessary and potentially serious risk. This highlights the inventive nature of the present innovation since it provides a way to extract DNA with solid phase materials that can be removed entirely from the AAV capsid preparation after processing.

The buffer for the concentrating TFF step is formulated to facilitate DNA digestion by a selected DNase enzyme in a subsequent process step. The selected DNase enzyme is added and allowed to incubate for a suitable length of time, with two notable differences from the routine practice of DNA lysis. 1) The smaller volume, due to enablement of TFF concentration, requires less enzyme. 2) Removal of inhibitors and buffer exchange into optimal lysis conditions for the selected DNase enzyme achieves much more effective reduction than the standard practice of simply adding it to raw cell culture harvest or lysate.

The porosity of the TFF membrane benefits from being the largest possible that still retains the capsids. Porosity ratings vary somewhat among manufacturers and among membrane materials but as a general matter, membranes with a molecular weight cutoff of 250 kDa to 300 kDa are ideal. Lower MWCO values support less effective clearance of contaminants but still substantially reduce contaminant content of the treated preparation. Overall, TFF membranes may be employed with MWCO ratings of 10 kDa to 300 kDa, 30 kDa to 300 kDa, 60 kDa to 300 kDa, 100 kDa to 300 kDa, 200 kDa to 300 kDa, or 250 kDa to 300 kDa, but preferably above 100 kDa. TFF membranes are available commercially in a range of physical formats and sizes to accommodate the various needs of R&D, scale-up, and manufacturing. They include flat membranes, singly or in stacks or rolls; and hollow fibers, singly or in bundles.

The technique of tangential flow filtration (TFF) is commonly performed in any one or any combination of three principle modes, as in the method of the invention. The first of those modes is concentration, where the objective is to remove excess fluid to make subsequent processing operations more efficient. The second mode is diafiltration. During diafiltration, the original fluid, salts, sugars, and buffer components, are filtered out while new fluid lacking those components is introduced. This has the effect of "buffer exchanging" the fluid in which the sample resides for the purpose of preparing the sample for some subsequent treatment. The term diafiltration is a combination of the two words "dialysis" and "filtration". The third mode is size-based purification. Concentration and diafiltration can be performed using tangential flow filtration membranes of any porosity that retains the product of interest, including membranes with molecular weight cutoffs of 10 kDa or less. The use of TFF as purification tool represents an extension where large-pore membranes are employed specifically to permit the elimination of biomolecule contaminants smaller than the product of interest. With the proviso that the porosity must be sufficiently small to retain the product of interest, the membrane with the largest pores that fulfil that requirement provide the benefit of removing the broadest size range of contaminating biomolecules. In the present case, since AAV capsids are fairly large on the scale of biological products, very large pore membranes, such as with molecular weight cutoffs up to 300 kDa, permit the elimination of the majority of proteins and other biomolecules. The smaller those contaminating biomolecules, the more effectively they are eliminated. This makes the technique especially well-suited for removal of DNase enzyme after DNA lysis, and for removal of histone proteins liberated from host cell DNA by lysis of the DNA they were previously associated with.

Many DNase enzymes are available commercially, each requiring its own unique conditions for best results. Preliminary experimental data indicate that so-called salt tolerant DNase enzymes may provide superior results. Some DNase enzymes also lyse RNA, which may be useful to the extent that RNA stabilizes large DNA heteroaggregates. RNase enzymes may be added to the DNase enzyme if the DNase enzyme lacks this ability. However, the ability of the lysis formulation to digest RNA is not obligatory to perform the method of the invention.

After lysis, the sample is treated again by TFF to remove the lysis enzymes, to remove DNA fragments, and to remove host histone proteins liberated by lysis of host DNA.

A particularly unexpected experimental finding is that lysis of DNA is not sufficient to completely solve the problem, even when lysis conditions are fully optimized. Experimental data suggest that residual histones interfere with chromatography methods, compromising their ability to remove contaminants, including DNA. Removing histones in advance of chromatography enhances removal of all contaminant classes and further enhances removal of contaminating DNA.

The second TFF step employs a buffer representing roughly physiological conditions, such as a pH between 6.5 and 7.5, and a sodium chloride concentration of 25-200 mM. Maintenance of roughly physiological conditions is believed to be a valuable contributory element to the enhancements provided by the method of the invention.

Sample preparation by TFF most often employs a buffer close to the binding conditions for the following chromatography method. Roughly physiological conditions are suitable for AAV capture by affinity chromatography, but the method of the invention reduces the overall contaminant load to such a degree that alternative methods such as capture by cation exchange chromatography or anion exchange chromatography can produce highly purified AAV without recourse to affinity chromatography. This is important because commercially available affinity chromatography media require low flow rates that increase process time and they cannot tolerate cleaning with 1.0 M NaOH. Capture with ion exchangers enables the use of chromatography media that supports high flow rates, higher product-binding capacity, and prolonged cleaning-sanitization with 1.0 M NaOH.

In one embodiment, the previous method steps are used to prepare the sample for loading to an affinity chromatography column.

In another embodiment, the previous method steps are used to prepare the sample for loading onto a positively charged chromatography column such as an anion exchanger. In this case, the sample will be diluted after the second TFF step so that its conductivity is sufficient low to ensure high capacity binding of AAV to the column. Its pH may also be adjusted to a higher value to support better discrimination of empty and full capsids. Magnesium and/or calcium salts may be added to modify the selectivity of empty-full separation. This approach may be appropriate for producing research-grade full capsids.

In another embodiment, the previous method steps are used to prepare the sample for loading onto a cation exchanger. The usual preparation to prepare a sample to be bound to a cation exchanger involves titrating the pH of the sample down to pH 3.5. This changes the titration states of the amino acid residues on the capsid protein surfaces, causing the capsid surface to become net electropositive. Their positive surface charge causes them to bind to bind to the negatively charged surface of the cation exchanger. However, it also has an unfavorable effect. By making the capsid surface electropositive it invites strong binding of DNA, or if DNA is already bound to the capsid exterior, low pH stabilizes that association. Either way, the low binding pH encourages DNA-binding to the capsid exterior.

Another aspect of this issue makes it yet more problematical. If DNA is bound to the capsid exteriors, excess histone proteins in the system have a very high probability of binding that capsid exterior-associated DNA. This highlights the value of the second TFF step, not only to remove DNA fragments but also histone proteins. In this context, it is important to emphasize that extremely low pH such as pH 3.5 also amplifies the positive charge on histone proteins, as well as other DNA binding proteins like transcription factors, and even ubiquitin.

The method of the invention includes another feature to address these potentials that also produces a surprising effect. Instead of equilibrating the sample to pH 3.5, the sample is equilibrated to a more moderate pH of 5.0 ± 0.25 in preparation for binding to the cation exchanger. The cation exchanger is equilibrated to the same conditions and the sample is loaded. After the sample is bound, the column is re-equilibrated to pH 3.5 and the AAV is eluted with an increasing salt gradient. Experimental data shows that this approach substantially reduces contamination of the AAV fraction eluting from the cation exchanger. It particularly removes small contaminants in the size range of DNA-binding proteins. This tends to suggest that histones and other DNA-binding proteins still in the sample after the previous steps of the method of the invention bind to the cation exchanger when the sample is applied to the column and they remain bound there while the AAV is eluted. This is consistent with the known behavior of histone proteins, which do not elute from cation exchangers in NaCl, and instead require guanidine or NaOH for elution. Whatever the mechanism, it substantially improves the quality of the eluted AAV.

After an initial cation exchange chromatography step, the AAV is applied to a positively charged chromatography device such as an anion exchanger to further extract DNA and separate empty capsids from full capsids, referring to capsids that contain the desired therapeutic DNA plasmid. AAV capsids may be similarly treated after an initial affinity chromatography step.

Cation exchange chromatography media are widely available commercially with a variety of different ligands and in range of different physical formats. The key defining feature of cation exchangers is that they are negatively charged. Biological species with a sufficiently positive charge bind to them, after which they can be eluted, most commonly by increasing the concentration of salt. However, all cation exchangers are multimodal, meaning that electrostatic interactions represent only one of the chemical mechanisms that contributes to the binding of biomolecules. The two most common cation exchange ligands are caboxy-alkane groups like carboxy-methyl, carboxy-ethyl, and carboxy-propyl groups; and sulfo-alkane groups like sulfo-methyl, sulfoethyl, and sulfopropyl groups. Carboxy-alkane cation exchange groups contain a carbonyl oxygen atom with two free lone pairs of electrons and a carboxyl oxygen atom with three free lone pairs of electrons. These lone pairs of electrons are hydrogen acceptors that endow these ligands with the potential to participate in hydrogen bonds. Sulfo-alkane cation exchange groups contain three oxygen atoms, two of which are uncharged but carry two lone pairs of free electrons. The third, which carries the negative charge, has three lone pairs of electrons that make the ligand a hydrogen donor and give it the ability to participate in hydrogen bonds. Cation exchangers sometimes employ alkane-phosphatidic acid residues. Depending on the pH, they have two negatively charge oxygen atoms each with three lone pairs of electrons and one uncharged oxygen atom with two lone pairs of electrons, or they have one negatively charged oxygen atom with three lone pairs and two uncharged oxygen atoms each with two lone pairs. In addition, phosphatidic acid based cation exchangers have the ability to participate in metal coordination bonding. Di-carboxy, tri-carboxy and polycarboxy cation exchange ligands also have the ability to bind metals. All of the above ligands also have varying levels of hydrophobic character depending on whether the associated alkane contains one, two, three or more carbon residues. Some cation exchange ligands also include hydrophobic rings. Thus for the purpose of the invention, cation exchangers are defined as chromatographic solid phases bearing negative charges, regardless of whatever other chemical reactivities they may embody. Cation exchanger are available commercially from multiple suppliers worldwide.

The same general logic applies to anion exchange chromatography media. For the purpose of the invention they are defined as chromatographic solid phases that bear positive charges, notwithstanding any additional chemical reactivities that they may embody. The charged nitrogen atoms of weak anion exchangers possess a single lone pair of free electrons that makes them hydrogen acceptors. Hydrogen atoms bonded directly to the nitrogen group can act as hydrogen donors. The charged nitrogen atoms of strong anion exchangers lack the lone pair of free electrons. The alkane groups associated with nitrogen atom on strong anion exchanger and most weak anion exchanges confer hydrophobicity. Some employ other hydrophobic structures. Some anion exchangers employ polymer-based ligands that create other combinations of chemically reactive properties. All anion exchangers are multimodal. Anion exchangers are available commercially from multiple suppliers worldwide.

Ligands for affinity chromatography consist of peptides and proteins, often representing antibodies or substructures of antibodies, such as F(ab)'2 regions, or Fab regions, or single chain light chain derivatives (scFV), or other recombinant structures, all of which mimic the specificity of antibodies. As with ion exchangers, many commercial variations are available from different suppliers worldwide.

The process of solid-phase DNA extraction begins when AAV-containing cell culture harvest or lysate is contacted with the positively charged surface(s) under roughly physiological conditions. The sample is contacted with the positively charged surface(s) for a period of about 1 minute to 4 hours.

In one embodiment where the positively charge surface(s) is in the form of particles, the volumetric proportion of particles to sample may be 0.1% to 10%, 1% to 5%, 2% to 5%, or a higher, lower, or intermediate proportion.

In one embodiment where the positively charge surface(s) is in the form of particles, the contact time may be 10 minutes to 240 minutes, or 20 minutes to 120 minutes, or 30 minutes to 60 minutes, or a higher, lower, or intermediate amount of time.

In one or more embodiments where the positively charged surface(s) is in the form of a flow-through device, the contact time may be 1 minute to 60 minutes, or 2 minutes to 30 minutes, or 5 minutes to 15 minutes, or a higher, lower, or intermediate amount of time.

In some cases, the positively charged particles may be mixed with particles of other chemical character. Experimental data show that a mixture of negatively charged particles with the positively charged particles removes more contaminants than positively charged particles alone.

DNase treatment may be performed for a period ranging from 15 minutes to 24 hours, or 30 minutes to 16 hours, or 1 hour to 8 hours, or a higher, lower, or intermediate amount of time. Since it is a particular objective to achieve the highest possible degree of DNA lysis, longer intervals such as 16 hours to 24 hours may be preferred.

DNase treatment is preferably performed within the same TFF unit that was used to concentrate and equilibrate the buffer for DNase treatment. Transmembrane pressure is set to zero and the sample is recirculated through the system after DNase addition to ensure thorough mixing. After the digestion process is complete, the TFF buffer is changed to a buffer with a pH of about 5 and a salt concentration of 20 mM to 100 mM. Transmembrane pressure is set to a positive value and TFF is recommenced. The majority of the enzyme is eliminated along with DNA fragments and most especially histone proteins.

The term pH of about 5 is understood to mean within a range of pH values from 4.0 to 6.0, or 4.5 to 5.5, or 4.75 to 5.25. It will be understood by persons of experience in the art that the lower the pH value, the higher the concentration of salt that may be present in the sample and still allow AAV to be captured by a cation exchanger. The sample may also be diluted at this point for the purpose of enhancing binding capacity of the cation exchanger. As a general matter, the visual clarity of the equilibrated sample will be an index of the suitability of the conditions. The appearance of turbidity or of whitish filaments or particles is an indication that the pH is excessively low.

With the AAV particles loaded onto the cation exchanger, the cation exchanger is re-equilibrated to a pH of about 3.5 ± 0.5, for example with a buffer containing formic acid, or acetic acid, or glycine, or some combination of those species at a concentration of 20 mM to 50 mM. This buffer may contain 20 mM to 200 mM NaCl to prevent binding by contaminants that are more weakly positive than AAV capsids. After re-equilibration, the column is eluted with a salt gradient to 1.0 M NaCl or more. After elution, the column is cleaned with 1.0 M NaOH, optionally including 1-3 M NaCl, and 20 mM to 50 mM EDTA.

Many commercial options are available for DNase enzymes to practice the method of the invention. Examples include endonuclease (Protean), Deoxyribonuclease (Worthington), Saltonase (Blirt), San-HQ and M-San HQ (ArcticZymes), Denarase (C-Lecta), TurboNuclease (Accelagen), Kaneka endonuclease (Kaneka), DNase (New England Biolabs), and Kryptonase (BIA Separations). Each of these enzymes has its own distinctive conditions to provide the most efficient digestion of DNA. Some are more tolerant of salt than others. Some are effective at lower temperatures than others. Some are able to lyse RNA as well as DNA. RNase enzymes can be added to those that lack this ability if desired.

Electropositive particles are available commercially in many forms from many suppliers. Particle based anion exchange chromatography media provide the majority of candidates, where the positive charge may derive from various forms and combinations of amine derivatives on the surface of the particles. Such forms and combinations include primary amine groups, individual residues and polymers such as polyallylamine and chitosan; secondary amine groups, individual residues and polymers; tertiary amine groups, individual residues and polymers such as DEAE and DEAE dextran; quaternary amine groups, individual residues and polymers such as Q, QA, and Q or QA dextran, or cholestyramine; individual ligands and polymers containing primary, secondary, and tertiary amine groups. Individual ligands that contain such combinations include immobilized ethylenediamine, which contains primary and secondary amine groups, and Tris(2-aminoethyl)amine, the immobilized form of which contains primary, secondary, and tertiary amine groups. Polymers of mixed amino forms include polyethyleneimine, which contains varying proportions of primary, secondary, and tertiary amines depending on the degree of branching within the polymer. The physical matrix of the particles maybe polymeric, silica-based, metal oxide-based, or combinations. The particles may be of any size, such as ranging from 0.1 µm to 200 µm. The particles may be non-porous or with porosity ranging from 0.1 nm 10 µm. The particles may be spherical, spheroid, aspheric, irregular, filamentous, or of mixed character.

The electropositive particles may be blended with other particles bearing other chemical reactivities. Preliminary experimental data indicate that a combination of electropositive (ethylenediamine) particles with electronegative (SO3) particles removes more protein contaminants in some cases that electronegative particles by themselves. Electropositive particles may also be multimodal as described above, where a given species of electropositive particles is able to interact with contaminants, not only through electrostatic interactions, but potentially through additional reactivities such as hydrogen bonding, hydrophobicity, or metal affinity.

Electropositive flow-through devices are also widely available commercially. An increasingly popular format includes so-called depth filters. Others potentially include simple membrane formats, also known as membrane adsorption units. Others include so-called hydrogels, columns of packed particles, columns of packed fibers, and devices that contain positively charged surfaces in multiple physical forms. The surface chemistry of such flow-through devices may include any of the surface chemistries described for particles.

### Examples

### Example 1

### Purification of AAV using the method of the invention

Beginning with a volume of 300 mL of cell lysate containing AAV serotype 8, 5 mL of positively charged particles was added. The particles were aspheric polymethacrylate particles in a size range of 20 µm to 40 µm and an average pore size of 2 µm, bearing ethylenediamine on their surfaces. Ethylenediamine immobilized on solid phase surfaces is positively charged by virtue of each residue bearing a primary amine and a secondary amine. The preparation was mixed for 30 minutes. The particles were removed by centrifugation at 10,000 x g for 10 minutes and filtration through a 0.45 µm PES membrane.

The particle-free supernatant was concentrated 14-fold by tangential flow filtration using a membrane with a 300 kDa molecular weight cut-off. Coincident with concentration it was buffer exchanged into 50 mM Tris, 0.5 M NaCl, 5 mM magnesium chloride, 1 % sucrose, 0.1 % poloxamer, pH 8.0.

Salt-tolerant DNase was added to a final concentration of 50 units per mL and the mixture was incubated for 16 hours at room temperature. Tangential flow filtration was recommenced, still in the same mechanical unit with the same original membrane into 20 mM HEPES, 30 mM sodium chloride, 1 % sucrose, 0.1 % poloxamer, pH 7.0.

The optically clear AAV-containing supernatant was titrated to pH 5.25 by gradual addition of 2 M acetic acid. A strong cation exchanger (SO3) was equilibrated to 20 mM MES, 30 mM sodium chloride, 1 % sucrose, 0.1 % poloxamer, pH 5.25. The equilibrated sample was loaded onto the equilibrated column, then washed with equilibration buffer to clear unbound contaminants.

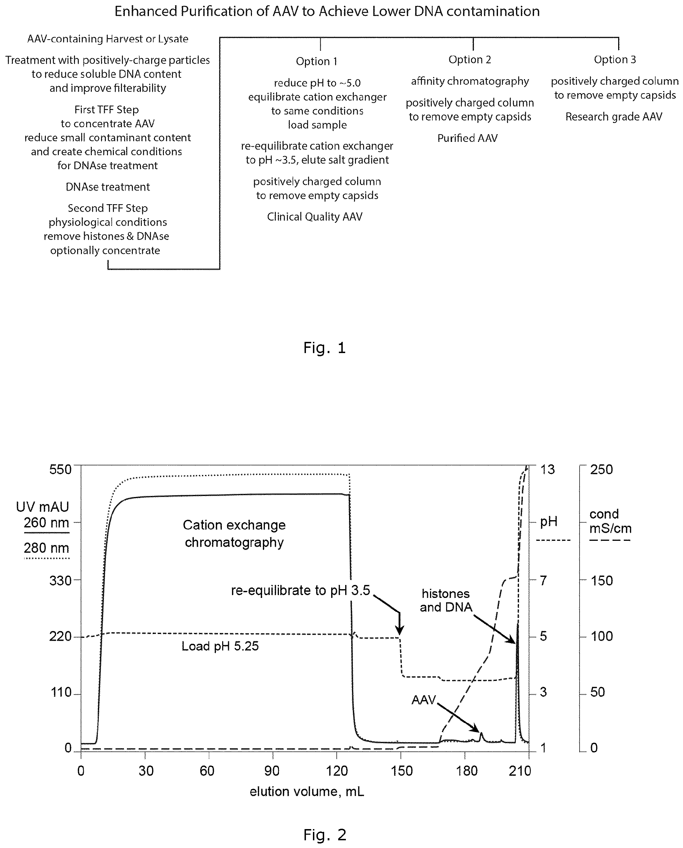

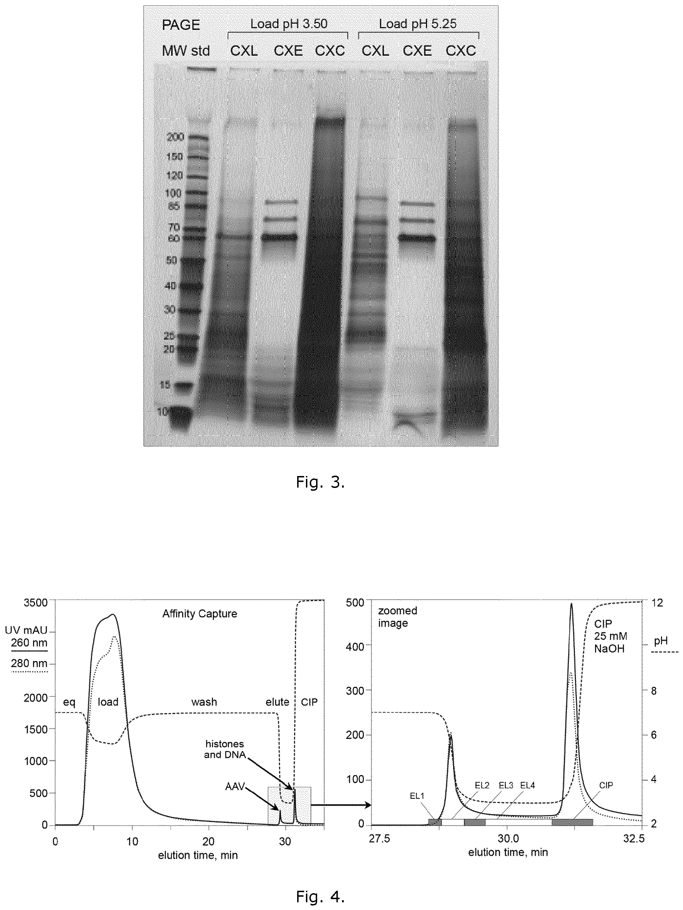

The cation exchange column was then re-equilibrated to 50 mM formic acid, 30 mM sodium chloride, 1 % sucrose, 0.1 % poloxamer, pH 3.5. Following re-equilibration, the column was eluted with a linear gradient of increasing sodium chloride, then cleaned with 1 M sodium hydroxide, 2 M sodium chloride. The chromatogram is shown in Figure 2. The AAV peak was evaluated by SDS-PAGE (Fig. 3).

### Example 2

### Purification of AAV using some of the steps of the method of the invention

A sample of the same material described in Example 1 was processed identically through the second TFF step. It was then titrated to pH 3.5 by gradual addition of 2 M formic acid.

An identical cation exchanger was equilibrated to 50 mM formic acid, 0.2 M sodium chloride, 1 % saccharose, 0.1 % poloxamer, pH 3.5. The sample was loaded, the column was washed with equilibration buffer, then eluted with the same linear gradient configuration as described in Example 1. The column was cleaned with 1 M NaOH, 2 M sodium chloride.

Figure 3 compares the degree of purification achieved in each of the two examples. The first 3 sample lanes illustrate results from example 2, in which the cation exchange step was conducted at pH 3.5. The second 3 sample lanes illustrate results from example 1, in which the cation exchanger and sample were equilibrated initially to pH 5.25, then after sample loading and washing at that pH, the column was re-equilibrated to pH 3.5 and eluted at that pH. The results show that the equilibrated sample contained less contamination at pH 5.25 enabling the cation exchanger to obtain a cleaner AAV fraction. The sample equilibrate to pH 3.5 contained a higher contaminant load and cation exchange produced a more impure fraction.

### Example 3

An experimental control using affinity chromatography was run to demonstrate the potential benefits of employing the method of the invention for other AAV capture methods. An AAV affinity column was equilibrated with 50 mM phosphate, 100 mM NaCl, pH 7.2. The sample was equilibrated to the same pH, filtered, then loaded onto the column. The column was washed with equilibration buffer then eluted with a step to 50 mM glycine, pH 3.0. After elution, the column was cleaned with 25 mM sodium hydroxide.

The chromatogram is illustrated in Figure 4. Comparison of the chromatogram in Figure 2 with chromatogram in Figure 4 shows that affinity chromatography has a problem that is similar to cation exchange chromatography regarding the binding of excess contaminants.

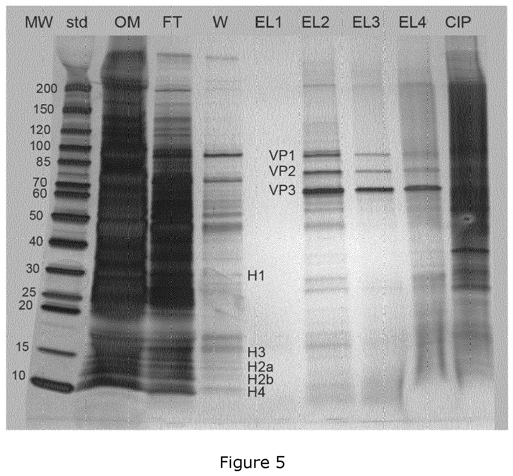

Results from analysis by SDS-PAGE are illustrated in Figure 5. Comparison of the PAGE gel in Figure 3 with chromatogram in Figure 5 shows that that method of the invention with cation exchange chromatography provides better purification than affinity chromatography without the benefit of the method of the invention.

Affinity chromatography is generally considered to provide the best capture-purification of all chromatography methods. The ability of the method of the invention to enable a general purification method like cation exchange chromatography to obtain clearly superior purification emphasizes the ability of the method of the invention to enhance purification potential. It also points out that it would substantially improve the purification performance of affinity chromatography as a capture method, and in some cases it would enable purification of at least research-grade AAV by following the initial steps of the method, as described in Example 2, with purification by anion exchange chromatography.

### Example 4

### Adaptation of the method to initial capture by affinity chromatography

The steps of the invention through the second TFF step are performed but not the subsequent steps of reducing the pH and applying the sample to a cation exchanger. Instead, the sample is applied directly to an affinity chromatography column. The column is then washed and eluted as it would be without the enhancements of the invention.

### Example 5

Adaptation of the method to initial capture by anion exchange chromatography.

The steps of the invention through the second TFF step are performed but not the subsequent steps of reducing the pH and applying the sample to a cation exchanger. Instead, the sample is diluted to a sufficiently low conductivity (salt concentration) to cause the AAV to bind to an anion exchanger equilibrated to the same conditions. The column is then washed and eluted as it would be without the enhancements of the invention.

### Example 6

Substitution of positively charged particles with positively charged internal surfaces of flow-through devices.

Any of the above examples can be practiced by substituting the positively charged particles with a large positively charged surface. Such surfaces are found in many commercial filtration media, such as depth filters.

## Claims

1. A method for reducing a contaminating DNA content of a preparation containing AAV capsids and contaminating DNA, comprising the steps of
a) Performing an extraction of DNA with a solid phase bearing positive charges at its surface said solid phase is contacted with the preparation at a pH of 7.0 ± 1.0, and a salt concentration of 10 mM to 200 mM yielding a first fraction,
b) Diafiltering the first fraction by a first tangential flow filtration to obtain a second fraction,
c) Treating the second fraction with DNase,
d) Diafiltering the DNase treated second fraction obtained by step c) by a second tangential flow filtration to a buffer with pH of 7.0 ± 1.0, and a salt concentration of 10 mM to 20 mM to yield a third fraction, and optionally
e) Concentrating the third fraction by tangential flow filtration before supplemental chromatography.

2. The method of claim 1 wherein the solid phase is in the form of loose bulk particles or a flow-through device.

3. The method of claim 1 or 2 wherein the tangential flow filtration is performed with membranes selected from the group consisting of hollow-fiber membranes, including single hollow fiber membranes or membranes in bundles; or flat membranes, including single membranes or membranes that are stacked or rolled into cylinders.

4. The method of any one of the claims 1 to 3 wherein the first filtration is performed in a buffer which is formulated to facilitate DNA digestion by a DNase.

5. The method of any one of the claims 3 or 4 wherein the tangential flow filtration membrane has a porosity with a molecular weight cutoff in the range of 10 kDa to 300 kDa, or 30 kDa to 300 kDa, or 60 kDa to 300 kDa, or 100 kDa to 300 kDa, or 200 kDa to 300 kDa, or 250 kDa to 300 kDa, preferably with molecular weight cutoffs of 100 kDa or more.

6. The method of any one of the claims 1 to 5 wherein the preparation containing AAV capsids and contaminating DNA contains AAV particles selected from the group consisting of AAV serotype 1, 2, 3, 4, 5, 6, 7, 8, 9, 10, 11, or other serotypes, including recombinant serotypes with features of more than one serotype.

7. The method of any one of the claims 1 to 6 wherein after step d) or step e) at least one of the following purification steps is employed:
(i) A cation exchange chromatography step or,
(ii) An affinity chromatography step or,
(iii) An anion exchange chromatography step.

8. The method of claim 7 wherein in purification step (i) the fraction of step d) or e) is loaded on a cation exchanger chromatography material at a pH of 4 to 6, in particular about pH 5, followed by
- a re-equilibration of the cation exchange chromatography material to about pH 3.5 ± 0.5,
- an elution is performed with a salt gradient, and
- an anion exchange chromatography treatment is performed thereafter.

9. The method of claim 7 wherein in purification step (ii) the fraction of step d) or e) is loaded on an affinity chromatography material and after elution an anion exchange chromatography treatment is performed.

10. The method of claim 7 wherein in purification step (iii) the fraction of step d) or e) is subjected to an anion exchange chromatography.

## Patentansprüche

1. Verfahren zum Vermindern eines kontaminierenden DNA-Gehalts eines Präparats, das AAV-Kapside und kontaminierende DNA enthält, umfassend die folgenden Schritte:
a) Durchführen einer Extraktion von DNA mit einer festen Phase, die positive Ladungen an ihrer Oberfläche trägt, wobei die feste Phase mit dem Präparat bei einem pH-Wert von 7,0 ± 1,0 und einer Salzkonzentration von 10 mM bis 200 mM kontaktiert wird, wodurch eine erste Fraktion erzeugt wird,
b) Diafiltrieren der ersten Fraktion durch eine erste Tangentialflussfiltration, derart dass eine zweite Fraktion erhalten wird,
c) Behandeln der zweiten Fraktion mit DNase,
d) Diafiltrieren der mit DNase behandelten zweiten Fraktion, die vom Schritt c) erhalten wurde, durch eine zweite Tangentialflussfiltration zu einem Puffer mit einem pH-Wert von 7,0 ± 1,0 und einer Salzkonzentration von 10 mM bis 20 mM, derart dass eine dritte Fraktion erhalten wird, und wahlweise
e) Konzentrieren der dritten Fraktion durch Tangentialflussfiltration vor zusätzlicher Chromatographie.

2. Verfahren nach Anspruch 1, wobei die feste Phase in der Form loser Massenpartikel oder einer Durchflussvorrichtung vorliegt.

3. Verfahren nach Anspruch 1 oder 2, wobei die Tangentialflussfiltration mit Membranen durchgeführt wird, die ausgewählt werden aus der Gruppe, die besteht aus Hohlfasermembranen, die einzelne Hohlfasermembranen oder Membranen in Bündeln umfassen; oder flachen Membranen, die einzelne Membranen oder Membranen umfassen, die gestapelt oder in Zylinder gewickelt sind.

4. Verfahren nach einem der Ansprüche 1 bis 3, wobei die erste Filtration in einem Puffer durchgeführt wird, der derart formuliert ist, dass DNA-Verdau durch DNase erleichtert wird.

5. Verfahren nach einem der Ansprüche 3 oder 4, wobei die Tangentialflussfiltrationsmembran eine Porosität mit einer Molekulargewicht-Trenngrenze im Bereich von 10 kDa bis 300 kDa oder 30 kDa bis 300 kDa oder 60 kDa bis 300 kDa oder 100 kDa bis 300 kDa oder 200 kDa bis 300 kDa oder 250 kDa bis 300 kDa, vorzugsweise mit Molekulargewicht-Trenngrenzen von 100 kDa oder höher, aufweist.

6. Verfahren nach einem der Ansprüche 1 bis 5, wobei das Präparat, das AAV-Kapside und kontaminierende DNA enthält, AAV-Partikel enthält, die ausgewählt sind aus der Gruppe, die besteht aus dem AAV-Serotyp 1, 2, 3, 4, 5, 6, 7, 8, 9, 10, 11 oder anderen Serotypen, die rekombinante Serotypen mit Merkmalen von mehr als einem Serotyp umfassen.

7. Verfahren nach einem der Ansprüche 1 bis 6, wobei nach dem Schritt d) oder dem Schritt e) mindestens einer der folgenden Reinigungsschritte eingesetzt wird:
(i) ein Kationenaustauschchromatographieschritt oder,
(ii) ein Affinitätschromatographieschritt oder,
(iii) ein Anionenaustauschchromatographieschritt.

8. Verfahren nach Anspruch 7, wobei im Reinigungsschritt (i) die Fraktion von Schritt d) oder e) auf ein Kationenaustauscherchromatographiematerial bei einem pH-Wert von 4 bis 6, insbesondere einem pH-Wert von etwa 5, geladen wird, gefolgt von:
- einer Neuäquilibrierung des Kationenaustauschchromatographiematerials auf einen pH-Wert von etwa 3,5 ± 0,5,
- einer Elution, die mit einem Salzgradienten durchgeführt wird, und
- einer Anionenaustauschchromatographiebehandlung, die danach durchgeführt wird.

9. Verfahren nach Anspruch 7, wobei im Reinigungsschritt (ii) die Fraktion von Schritt d) oder e) auf ein Affinitätschromatographiematerial geladen wird und nach der Elution eine Anionenaustauschromatographiebehandlung durchgeführt wird.

10. Verfahren nach Anspruch 7, wobei im Reinigungsschritt (iii) die Fraktion von Schritt d) oder e) einer Anionenaustauschchromatographie unterzogen wird.

## Revendications

1. Procédé pour réduire la teneur en ADN contaminant d'une préparation contenant des capsides d'AAV et de l'ADN contaminant, comprenant les étapes de
a) mise en oeuvre d'une extraction d'ADN avec une phase solide portant des charges positives à sa surface, laquelle phase solide est mise en contact avec la préparation à un pH de 7,0 ± 1,0, et à une concentration de sel de 10 mM à 200 mM, ce qui donne une première fraction,
b) diafiltration de la première fraction par une première filtration à écoulement tangentiel pour que soit obtenue une deuxième fraction,
c) traitement de la deuxième fraction avec une DNase,
d) diafiltration de la deuxième fraction traitée à la DNase obtenue dans l'étape c) par une deuxième filtration à écoulement tangentiel contre un tampon ayant un pH de 7,0 ± 1,0, et à une concentration de sel de 10 mM à 20 mM pour engendrer une troisième fraction, et éventuellement
e) concentration de la troisième fraction par filtration à écoulement tangentiel avant chromatographie supplémentaire.

2. Procédé selon la revendication 1, dans lequel la phase solide est sous la forme de particules en vrac non tassées ou d'un dispositif à écoulement traversant.

3. Procédé selon la revendication 1 ou 2, dans lequel la filtration à écoulement tangentiel est effectuée avec des membranes choisies dans le groupe constitué par les membranes à fibres creuses, y compris les membranes à une seule fibre creuse ou les membranes en faisceaux ; ou des membranes plates, y compris des membranes uniques ou des membranes qui sont empilées ou enroulées en cylindres.

4. Procédé selon l'une quelconque des revendications 1 à 3, dans lequel la première filtration est effectuée dans un tampon qui est formulé pour faciliter la direction d'ADN par une DNase.

5. Procédé selon l'une quelconque des revendications 3 et 4, dans lequel la membrane de filtration à écoulement tangentiel a une porosité avec une séparation des masses moléculaires située dans la plage allant de 10 kDa à 300 kDa, ou de 30 kDa à 300 kDa, ou de 60 kDa à 300 kDa, ou de 100 kDa à 300 kDa, ou de 200 kDa à 300 kDa, ou de 250 kDa à 300 kDa, de préférence avec une séparation des masses moléculaires de 100 kDa ou plus.

6. Procédé selon l'une quelconque des revendications 1 à 5, dans lequel la préparation contenant des capsides d'AAV et de l'ADN contaminant contient des particules d'AAV choisies dans le groupe constitué par les sérotypes d'AAV 1, 2, 3, 4, 5, 6, 7, 8, 9, 10, 11, ou d'autres sérotypes, y compris les sérotypes recombinés ayant des caractéristiques de plusieurs sérotypes.

7. Procédé selon l'une quelconque des revendications 1 à 6, dans lequel, après l'étape d) ou l'étape e), au moins une des étapes de purification suivantes est employée :
(i) une étape de chromatographie par échange de cations ou
(ii) une étape de chromatographie par affinité ou
(iii) une étape de chromatographie par échange d'anions.

8. Procédé selon la revendication 7, dans lequel, dans l'étape de purification (i), la fraction de l'étape d) ou e) est chargée sur un matériau de chromatographie échangeur de cations à un pH de 4 à 6, en particulier à un pH d'environ 5, opération suivie de
- un rééquilibrage du matériau de chromatographie par échange de cations à un pH d'environ 3,5 ± 0,5,
- la mise en oeuvre d'une élution avec un gradient de sel, et
- la mise en oeuvre ensuite d'un traitement de chromatographie par échange d'ions.

9. Procédé selon la revendication 7, dans lequel, dans l'étape de purification (ii), la fraction de l'étape d) ou e) est chargée sur un matériau de chromatographie par affinité et, après l'élution, un traitement de chromatographie par échange d'anions est effectué.

10. Procédé selon la revendication 7, dans lequel, dans l'étape de purification (iii), la fraction de l'étape d) ou e) est soumise à une chromatographie par échange d'anions.
